# EUROPEAN PATENT SPECIFICATION

(11) **EP 4 278 358 B1**
(45) Date of publication and mention of the grant of the patent: **08.07.2026**
(21) Application number: 22704028.4
(22) Date of filing: 11.01.2022
(51) Int. Cl.: G16H 20/17, G16H 40/63, G16H 40/67, G16H 40/60, A61M 5/142, A61M 5/168, A61M 5/172

(54) **SYSTEM AND METHOD FOR REDUCED INFUSION ADMINISTRATION LINE ERROR**
SYSTEM UND VERFAHREN FÜR REDUZIERTE INFUSIONSVERWALTUNGSLEITUNGSFEHLER
SYSTÈME ET PROCÉDÉ POUR UNE ERREUR DE LIGNE D'ADMINISTRATION DE PERFUSION RÉDUITE

(30) Priority: 12.01.2021 US 202163136609 P
(43) Date of publication of application: 22.11.2023
(73) Proprietor: CareFusion 303, Inc., San Diego, CA 92130 (US)
(72) Inventor: WORKMAN, Michael K., San Diego, California 92130 (US); DIGGETT, Lisa, San Diego, California 92130 (US); KNIGHT, Claire Ellen, San Diego, California 92130 (US); COLLINS, Laura Ann, San Diego, California 92130 (US)
(74) Representative: Zenz Patentanwälte Partnerschaft mbB
(86) International application number: PCT/US2022/012046
(87) International publication number: WO 2022/155157

(56) References cited:
- US-A1- 2002 038 392
- US-A1- 2005 277 873
- US-A1- 2007 233 520
- US-A1- 2020 402 651

## Description

This application claims the benefit of U.S. Provisional Application Serial No. 63/136,609, entitled "SYSTEM AND METHOD FOR REDUCED INFUSION ADMINISTRATION LINE ERROR," filed on January 12, 2021.

### TECHNICAL FIELD

This application relates generally to reducing infusion administration line error.

### BACKGROUND

Medical devices such as infusion devices are used to administer medical fluids to patients. An infusion administration line error may occur when an infusion container housing the medical fluid is not correctly associated to an infusion administration set and/or the infusion administration set is not correctly associated to an infusion pump. When infusion order parameters recorded in an electronic medical records storage and retrieval system is sent to a different infusion pump from that which is intended to carry out the infusion, the medical fluid may not be infused at the correct flow rate or other infusion parameters.

In US 2002 038 392 A1, a method and apparatus are described for controlling and monitoring the delivery of intravenous (IV) medication. This involves the use of information tags on IV bags to specify delivery parameters, obtaining these parameters, associating a controller with a specific patient, and then comparing the patient's information with the delivery parameters. Based on this comparison, it determines the appropriateness of the medicant delivery to the patient and adjusts pump control accordingly.

US 2007 233 520 A1 outlines a system, method, and computer program designed to program a medical device for administering medication to a patient. This setup includes the medical device itself, a scanner linked to a point of care system, and a medication management unit. A computer within the POC system is capable of directly programming the medical device with MMU authorization, following a safety check.

US2005277873 Al discloses a medical delivery system with a line set associated with a medication container, the line set being associated with an identifier comprising information for configuring the medical delivery system.

### SUMMARY OF THE INVENTION

The invention is set out in the appended claims

### SUMMARY OF RELATED DISCLOSURE

Correctly associating an infusion container to an infusion administration set, and an infusion pump can increase patient safety by ensuring that the correct drug is infused to a patient at the correct flow rate and for the correct duration. Similarly, medical record keeping for the patient can also be more seamlessly carried out when the electronic medical records storage and retrieval system is able to verify that the correct patient is receiving the correct drug using a particular administration set that is loaded into the correct infusion pump. Manually checking for infusion administration line error may be time-consuming, and can cause user frustration, which may compromise patient safety if an infusion administration line error is not timely detected.

For example, some association methods use pressure changes (e.g., a caregiver squeezing on the infusion container and detecting a change in pressure downstream in an infusion line of the administration set) or other additional steps to confirm the proper bag-set-pump associational relationship. Such manual confirmation methods may benefit from the systems, devices, and methods described herein that provide an automated, more intuitive, and visual way to confirm the proper associational relationship is in place prior to starting an infusion.

Accordingly, there is a need for systems, devices, and methods that help ensure that the infusion container, the infusion administration set, and the infusion pump are correctly associated.

Part of the infusion preparation process may include the caregiver associating an infusion order recorded at the electronic medical records storage and retrieval system to (1) a fluid container (e.g., bags, bottles, etc.) including the medication to be infused, (2) an administration set used to infuse the container of medication, and (3) an infusion pump that performs the infusion process. Associating the infusion order may include using a scanner to scan a unique identifier (e.g., a 2D barcode) associated with the infusion fluid container of medication, and using the scanner to scan a unique identifier associated with the administration set.

The disclosed devices, systems and methods may adjust the infusion pump in response to detecting an incorrect association between the container and pumping module. The adjustment may include activating a perceivable indicator, adjusting a display or other user interface, adjusting power supply or mode, stopping or slowing a motor, or the like. For example, the adjustment may cause the pump to generate an alert indicating that an incorrect association is present. As an additional or alternative adjustment, the adjustment may cause the pump to prohibit operation such as preventing initiation of the infusion until the correct association between the infusion fluid container, the administration set, and the infusion pump is made. This can help automate the initiation of the infusion process such that only infusion processes without any infusion administration line errors are permitted to proceed.

The disclosed subject matter also relates to a method of associating a fluid container, an administration set, and a pump of an infusion device includes receiving, at an infusion device, an indication that an administration set is being coupled to a pump, the indication including an administration set identifier of the administration set. The method includes transmitting to an electronic medical records storage and retrieval system, data about an association of an infusion container identifier with the administration set identifier. The method includes receiving, from the electronic medical records storage and retrieval system, first information about a content of the infusion container associated with the infusion container identifier. The method includes receiving, at the infusion device, a drug identification for a medical fluid to be infused. The method includes evaluating, at the infusion device, the drug identification with the first information about the content of the infusion container. In accordance with a determination that the first information about the content of the infusion container matches the drug identification: permitting the pump to start infusing the content of the infusion container. And in accordance with a determination that the first information about the content of the infusion container does not match the drug identification of the medical fluid received at the pump: preventing the pump from infusing the content of the infusion container; and generating an alert indicating that the content of the infusion container does not match the drug identification received at the infusion device. The method may be implemented using a system that includes one or more processors and a memory including instructions that, when executed by the one or more processors, cause the one or more processors to perform the steps of the method described herein.

Other aspects include corresponding apparatus, and computer program products for implementation of the corresponding system and its features.

It is understood that other configurations of the subject technology will become readily apparent to those skilled in the art from the following detailed description, wherein various configurations of the subject technology are shown and described by way of illustration. As will be realized, the subject technology is capable of other and different configurations and its several details are capable of modification in various other respects, all without departing from the scope of the subject technology. Accordingly, the drawings and detailed description are to be regarded as illustrative in nature and not as restrictive.

### BRIEF DESCRIPTION OF THE DRAWINGS

For a better understanding of the various described implementations, reference should be made to the Description below, in conjunction with the following drawings. Like reference numerals refer to corresponding parts throughout the figures and description.
FIG. 1 depicts an example of an institutional patient care system of a healthcare organization, according to aspects of the subject technology.
FIG. 2 depicts an example of a system for reducing infusion administration line errors, according to aspects of the subject technology.
FIG. 3A depicts a first example workflow 300, according to aspects of the subject technology.
FIG. 3B shows a second example workflow 350, according to aspects of the subject technology.
FIG. 4 depicts an example method for associating an infusion container, an administration set, and a pump of an infusion device, according to aspects of the subject technology.
FIG. 5 is a conceptual diagram illustrating an example electronic system for associating an infusion container, an administration set, and a pump of an infusion device, according to aspects of the subject technology.

### DESCRIPTION

Reference will now be made to implementations, examples of which are illustrated in the accompanying drawings. In the following description, numerous specific details are set forth in order to provide an understanding of the various described implementations. However, it will be apparent to one of ordinary skill in the art that the various described implementations may be practiced without these specific details. In other instances, well-known methods, procedures, components, circuits, and networks have not been described in detail so as not to unnecessarily obscure aspects of the implementations.

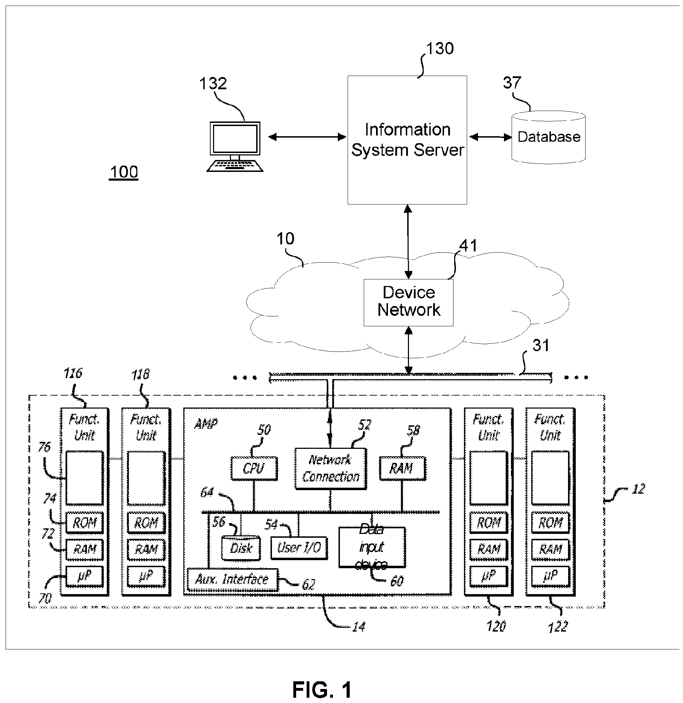

FIG. 1 depicts an example of an institutional patient care system 100 of a healthcare organization, according to aspects of the subject technology. In FIG. 1, a patient care device (or "medical device" generally) 12 is connected to a hospital network 10. The term patient care device (or "PCD") may be used interchangeably with the term patient care unit (or "PCU"), either which may include various ancillary medical devices such as an infusion pump, a vital signs monitor, a medication dispensing device (e.g., cabinet, tote), a medication preparation device, an automated dispensing device, a module coupled with one of the aforementioned (e.g., a syringe pump module configured to attach to an infusion pump), or other similar devices. Each patient care device 12 is connected to an internal healthcare network 10 by a transmission channel 31. Transmission channel 31 is any wired or wireless transmission channel, for example an 802.11 wireless local area network (LAN). In some implementations, network 10 also includes computer systems located in various departments throughout a hospital. For example, network 10 of FIG. 1 optionally includes computer systems associated with an admissions department, a billing department, a biomedical engineering department, a clinical laboratory, a central supply department, one or more unit station computers and/or a medical decision support system. As described further below, network 10 may include discrete subnetworks. In the depicted example, network 10 includes a device network 41 by which patient care devices 12 (and other devices) communicate in accordance with normal operations.

Additionally, institutional patient care system 100 may incorporate a separate information system server 130, the function of which will be described in more detail below. Moreover, although the information system server 130 is shown as a separate server, the functions and programming of the information system server 130 may be incorporated into another computer, if such is desired by engineers designing the institution's information system. Institutional patient care system 100 may further include one or multiple device terminals 132 for connecting and communicating with information system server 130. Device terminals 132 may include personal computers, personal data assistances, mobile devices such as laptops, tablet computers, augmented reality devices, or smartphones, configured with software for communications with information system server 130 via network 10.

Patient care device 12 comprises a system for providing patient care, such as that described in Eggers et al. Patient care device 12 may include or incorporate pumps, physiological monitors (e.g., heart rate, blood pressure, ECG, EEG, pulse oximeter, and other patient monitors), therapy devices, and other drug delivery devices may be utilized according to the teachings set forth herein. In the depicted example, patient care device 12 comprises a control module 14, also referred to as interface unit 14, connected to one or more functional modules 116, 118, 120, 122. Interface unit 14 includes a central processing unit (CPU) 50 connected to a memory, for example, random access memory (RAM) 58, and one or more interface devices such as user interface device 54, a coded data input device 60, a network connection 52, and an auxiliary interface 62 for communicating with additional modules or devices. Interface unit 14 also, although not necessarily, includes a main non-volatile storage unit 56, such as a hard disk drive or non-volatile flash memory, for storing software and data and one or more internal buses 64 for interconnecting the aforementioned elements.

In various implementations, user interface device 54 is a touch screen for displaying information to a user and allowing a user to input information by touching defined areas of the screen. Additionally or in the alternative, user interface device 54 could include any means for displaying and inputting information, such as a monitor, a printer, a keyboard, softkeys, a mouse, a track ball and/or a light pen. Data input device 60 may be a bar code reader capable of scanning and interpreting data printed in bar coded format. Additionally or in the alternative, data input device 60 can be any device for entering coded data into a computer, such as a device(s) for reading a magnetic strips, radio-frequency identification (RFID) devices whereby digital data encoded in RFID tags or smart labels (defined below) are captured by the reader 60 via radio waves, PCMCIA smart cards, radio frequency cards, memory sticks, CDs, DVDs, or any other analog or digital storage media. Other examples of data input device 60 include a voice activation or recognition device or a portable personal data assistant (PDA). Depending upon the types of interface devices used, user interface device 54 and data input device 60 may be the same device. Although data input device 60 is shown in FIG. 1 to be disposed within interface unit 14, it is recognized that data input device 60 may be integral within pharmacy system 34 or located externally and communicating with pharmacy system 34 through an RS-232 serial interface or any other appropriate communication means. Auxiliary interface 62 may be an RS-232 communications interface, however any other means for communicating with a peripheral device such as a printer, patient monitor, infusion pump or other medical device may be used without departing from the subject technology. Additionally, data input device 60 may be a separate functional module, such as modules 116, 118, 120 and 122, and configured to communicate with controller 14, or any other system on the network, using suitable programming and communication protocols.

Network connection 52 may be a wired or wireless connection, such as by Ethernet, WiFi, BLUETOOTH, an integrated services digital network (ISDN) connection, a digital subscriber line (DSL) modem or a cable modem. Any direct or indirect network connection may be used, including, but not limited to a telephone modem, an MIB system, an RS232 interface, an auxiliary interface, an optical link, an infrared link, a radio frequency link, a microwave link or a WLANS connection or other wireless connection.

Functional modules 116, 118, 120, 122 are any devices for providing care to a patient or for monitoring patient condition. As shown in FIG. 1, at least one of functional modules 116, 118, 120, 122 may be an infusion pump module such as an intravenous infusion pump for delivering medication or other fluid to a patient. For the purposes of this discussion, functional module 116 is an infusion pump module. Each of functional modules 118, 120, 122 may be any patient treatment or monitoring device including, but not limited to, an infusion pump, a syringe pump, a PCA pump, an epidural pump, an enteral pump, a blood pressure monitor, a pulse oximeter, an EKG monitor, an EEG monitor, a heart rate monitor or an intracranial pressure monitor or the like. Functional module 118, 120 and/or 122 may be a printer, scanner, bar code reader or any other peripheral input, output or input/output device.

Each functional module 116, 118, 120, 122 communicates directly or indirectly with interface unit 14, with interface unit 14 providing overall monitoring and control of device 12. Functional modules 116, 118, 120, 122 may be connected physically and electronically in serial fashion to one or both ends of interface unit 14 as shown in FIG. 1, or as detailed in Eggers et al. However, it is recognized that there are other means for connecting functional modules with the interface unit that may be utilized without departing from the subject technology. It will also be appreciated that devices such as pumps or patient monitoring devices that provide sufficient programmability and connectivity may be capable of operating as stand-alone devices and may communicate directly with the network without connected through a separate interface unit or control unit 14. As described above, additional medical devices or peripheral devices may be connected to patient care device 12 through one or more auxiliary interfaces 62.

Each functional module 116, 118, 120, 122 may include module-specific components 76, a microprocessor 70, a volatile memory 72 and a nonvolatile memory 74 for storing information. It should be noted that while four functional modules are shown in FIG. 1, any number of devices may be connected directly or indirectly to controller unit 14. The number and type of functional modules described herein are intended to be illustrative, and in no way limit the scope of the subject technology. Module-specific components 76 include any components necessary for operation of a particular module, such as a pumping mechanism for infusion pump module 116.

While each functional module may be capable of a least some level of independent operation, interface unit 14 monitors and controls overall operation of device 12. For example, as will be described in more detail below, interface unit 14 provides programming instructions to the functional modules 116, 118, 120, 122 and monitors the status of each module. The programming instructions may be based a volume or flow rate detected using at least some of the features described.

Patient care device 12 is capable of operating in several different modes, or personalities, with each personality defined by a configuration database. The configuration database may be a database 56 internal to patient care device, or an external database 37. A particular configuration database is selected based, at least in part, by patient-specific information such as patient location, age, physical characteristics, or medical characteristics. Medical characteristics include, but are not limited to, patient diagnosis, treatment prescription, medical history, medical records, patient care provider identification, physiological characteristics or psychological characteristics. As used herein, patient-specific information also includes care provider information (e.g., physician identification) or a patient care device's 10 location in the hospital or hospital computer network. Patient care information may be entered through interface device 52, 54, 60 or 62, and may originate from anywhere in network 10, such as, for example, from a pharmacy server, admissions server, laboratory server, and the like.

Medical devices incorporating aspects of the subject technology may be equipped with a Network Interface Module (NIM), allowing the medical device to participate as a node in a network. While for purposes of clarity the subject technology will be described as operating in an Ethernet network environment using the Internet Protocol (IP), it is understood that concepts of the subject technology are equally applicable in other network environments, and such environments are intended to be within the scope of the subject technology.

Data to and from the various data sources can be converted into network-compatible data with existing technology, and movement of the information between the medical device and network can be accomplished by a variety of means. For example, patient care device 12 and network 10 may communicate via automated interaction, manual interaction or a combination of both automated and manual interaction. Automated interaction may be continuous or intermittent and may occur through direct network connection 54 (as shown in FIG. 1), or through RS232 links, MIB systems, RF links such as BLUETOOTH, IR links, WLANS, digital cable systems, telephone modems or other wired or wireless communication means. Manual interaction between patient care device 12 and network 10 involves physically transferring, intermittently or periodically, data between systems using, for example, user interface device 54, coded data input device 60, bar codes, computer disks, portable data assistants, memory cards, or any other media for storing data. The communication means in various aspects is bidirectional with access to data from as many points of the distributed data sources as possible. Decision-making can occur at a variety of places within network 10. For example, and not by way of limitation, decisions can be made in health information server (HIS) 30, decision support 48, remote data server 49, hospital department or unit stations 46, or within patient care device 12 itself.

Direct communications with medical devices operating on a network in accordance with the subject technology may be performed through information system server 30, also known as the remote data server (RDS). In accordance with aspects of the subject technology, network interface modules incorporated into medical devices such as, for example, infusion pumps or vital signs measurement devices, ignore all network traffic that does not originate from an authenticated RDS. The primary responsibilities of the RDS of the subject technology are to track the location and status of all networked medical devices that have NIMs, and maintain open communication.

Flow rate refers to the volume of fluid that is displaced over time, and can be either instantaneous flow or cumulative flow. Instantaneous flow is the volume of flow over a fixed period of time. Cumulative flow is the cumulative value of flow from the start to the finish of the measurement. For example, a 500 mL of medication infused over a period of 8 hour, represents a cumulative flow rate of (500 mL /8 hour) 62.5 mL/hr. Flow rate accuracy performance is typically described by trumpet curves which display the flow continuity for both the instantaneous and cumulative flow rates.

The pump flow rate depends on factors such as tubing dimensions (e.g., inner diameter, length, material properties), mechanism speed (e.g., cam rotations per minute (RPM)), and tubing occlusion which is the amount of squeeze applied to the tubing to create a fluid seal. The pump flow rate is also dependent on the tubing wall thickness and occluder finger force. Nonconformance in any of these or other fault conditions can result in uncontrolled flow rate or volume delivered by the pump. Thus, there is a need to be able to verify the flow rate and volume delivered by the pump, present a notification of any aberrant condition, and, if possible, adjust one or more medical devices to ensure and enhance patient safety.

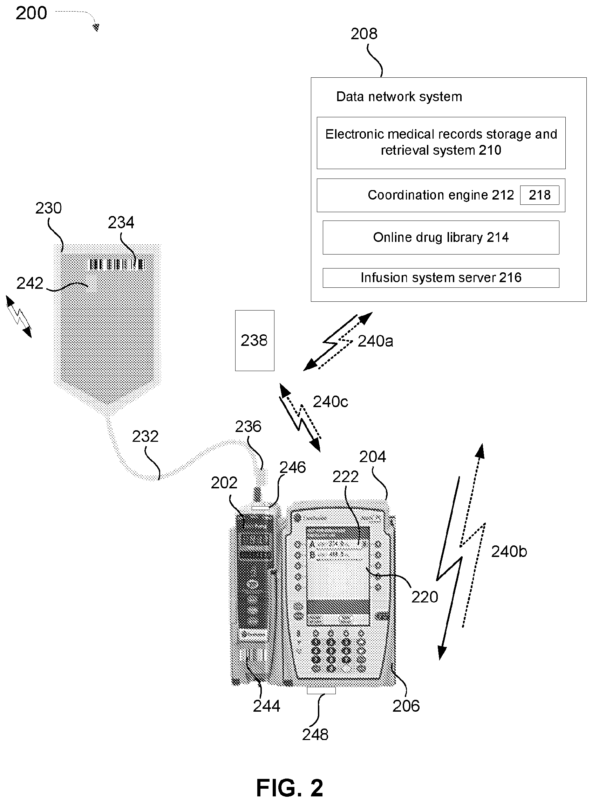

FIG. 2 depicts an example of a system for reducing infusion administration line errors, according to aspects of the subject technology. The system 200 includes an infusion pump 202 controlled by an infusion device 204. The infusion device 204 may control additional infusion pumps in addition to the infusion pump 202. In some implementations, the infusion device 204 includes a communication module 206 (e.g., a wireless communication module, a wired communication module). In some implementations, the communication module 206 of the infusion device 204 includes a network data transceiver.

An infusion container 230 houses a medication that is to be infused to a patient. The infusion container 230 includes a unique identifier that is associated to the infusion container 230. In some implementations, the infusion container may be an infusion container, and the identifier of the infusion container may be a bag identifier 234. In some implementations, the bag identifier 234 is a barcode or other optically scannable indicia that is affixed to the infusion container, and the barcode can be read or scanned by a scanner. In some implementations, a caregiver who prepares the infusion container 230 affixes the bag identifier 234 onto the infusion container 230 and provides information about the medication content (e.g., drug identification, drug concentration, bag volume) of the infusion container 230 to the data network system 208. A data record in the data network system associates the bag identifier 234 to the specific medication content within the infusion container 230. In this way, the data network system is also able to identify the medication administered to a patient simply by using the bag identifier to search for the data record that associates the bag identifier to the medication contained in the infusion container 230.

An administration set 232 is used to infuse the content of the infusion container 230 to a patient via the infusion pump 202. In some implementations, the administration set 232 includes a set identifier 236 that is attached to the administration set 232. The set identifier 236 may be included in a clamp of a flow stop (not shown in FIG. 2). The clamp may include electronic circuitry or a communication module (e.g., RF transmitter or RF tag) that transmits and receives communication data signals that provide identity information about the administration set 232. The clamp may be first associated with a tubing of the administration set to form the set identifier. In some implementations, the set identifier 236 may include a 2D barcode that can be read or scanned by a scanner.

The infusion system 200 includes an administration set receiver 246. The infusion pump may be configured to provide a force to at least a portion of the administration set 232 after the administration set is received by the administration set receiver 246. In some implementations, the administration set receiver 246 includes an administration set key channel for receiving an administration set key included in the administration set 232, and a sensor having a detection field of at least a portion of the administration set key channel. In some implementations, the set identifier 236 includes the administration set key. When the administration set 232 is loaded into the infusion pump 202, the infusion pump 202 may receive identifying information from the set identifier 236, allowing the infusion pump 202 to identify the administration set 232 that has been loaded. For example, detecting the administration set identifier 236 may include activating the sensor in the administration set receiver 246, and receiving a value from the sensor detected from the administration set key received by the administration set key channel. In some implementations, the sensor in the administration set receiver 246 is an optical sensor that is configured to scan a barcode that forms the administration set key. In some implementations, the sensor in the administration set receiver 246 is an electronic sensor that is configured to receive electrical control signals from the administration set key that identifies the administrate set 232. In some implementations, the administration set receiver 246 may be integrated within the infusion device 204 and/or be mechanically engaged with the administration set 232. In some implementations, the administration set receiver 246 is an add-on or standalone device separate from the infusion device 204 and is only optionally mechanically engaged with the administration set 232.

In some implementations, infusion pump 202 is also able to record how long the administration set 232 has been loaded into the infusion pump 202, and how long the administration set 232 is actively used to infuse medication to the patient. The infusion device 204 and/or the infusion pump 202 is able to generate an alert signal when the administration set 232 has exceeded the duration of use specified for the administration set (e.g., 24 hours, 48 hours, 72 hours).

According to various implementations, a scanner 238 (e.g., an optical scanning device) may be used to scan one or more of the bag identifier 234, the set identifier 236, or a unique identifier 244 affixed on the infusion pump 202. The scanner 238 may be a handheld scanner, or a mobile device having a camera or scanning capabilities. In some implementations, the scanner is able to receive an RF transmission/ communication signals from the set identifier 236, and retrieve identifying information about the administration set 232 from the communication signals. The scanner 238 is able to transmit the scanned information to the infusion device 204 via a communication connection 240c. In some implementations, the scanner transmits the scanned information directly to the data network system 208 using a communication connection 240a. In some implementations, the scanner 238 may transmit the scanned information to a workstation of the caregiver.

In some implementations, the infusion system 200 includes a wireless scanning device 248. The wireless scanning device 248 is able to detect and receive the infusion container identifier after the infusion container enters a scanning range scans of the wireless scanning device 248. For example, in some implementations, the wireless scanning device 248 emits a electromagnetic signal that is reflected by an infusion container identifier that is in the scanning range of the wireless scanning device. The reflected signal is then detected by the wireless scanning device 248, and the wireless scanning device 248 is able to provide the infusion container identifier to a processor that is configured to identify an association between the infusion container identifier and an administration set identifier. In some implementations, the wireless scanning device 248 may be integrated within the infusion device 204. In some implementations, the wireless scanning device 248 may be an add-on or standalone device separate from the infusion device 204. In some implementations, an RFID or other signaling device 242 is affixed to the infusion container 230. The RFID or signaling device 242 is able to receive a communication signal from the communication module 206 of the infusion device 204. The communication signal can activate the RFID or signaling device 242 to switch on an LED light on the signaling device 242.

The system 200 provides data connections between a data network system 208 and the infusion device 204. The data network system 208 may include an electronic medical records storage and retrieval system 210, a coordination engine 212, an online drug library 214, and an infusion system server 216. In some implementations, the coordination engine 212 includes an infusion adaptor 218. The infusion adaptor 218 provides a template to the coordination engine 212 to allow different preconfigured internal messaging protocols to be built for different infusion-specific applications of the infusion device 204.

The electronic medical records storage and retrieval system 210 (hereinafter "EMR system 210") stores, for a particular patient, infusion orders and other medications prescribed by a doctor to that particular patient. In addition, the EMR system 210 may also create auto-programming request (APR) messages that are delivered to the infusion device 204 to program the infusion pump 202 for a particular infusion order. In some implementations, the EMR system may include verification or infusion software and/or instructions that create the APR messages. In some implementations, these software and/or instructions may be a separate system on a different server and/or system.

In some implementations, the APR messages that are sent to the infusion device are routed through the coordination engine 212 and the infusion system server 216. In some implementations, the coordination engine 212 interacts with the infusion device 204 via the infusion adaptor 218. The infusion adaptor 218 is an application that interfaces between the infusion device 204 and the coordination engine 212. The coordination engine 212 performs various functions, including checking that the order information in the APR messages sent from the EMR system 210 to the infusion device 204 is complete and no required fields are missing or contain invalid data values.

In some implementations, the EMR system 210 receives a "start infusion request" command from the infusion device 204 and uses a unique identifier 244 (e.g., serial number of the infusion pump 202) scanned by the scanner and medication information entered by a caregiver to create an auto programming request (APR) message. In some implementations, a particular infusion pump lights up where the caregiver may load the administration set without the caregiver having to scan the unique identifier 224 associated with the infusion pump channel. In some implementations, when the set identifier 236 is not automatically detected by the infusion pump, the scanner 238 is controlled to expect a third scan of a unique identifier (e.g., 244) associated with the infusion pump 202. The EMR system 210 sends the correct infusion order parameters (e.g., fluid flow rate, infusion time, infusion volume, etc.) to the infusion device 204 (or the infusion pump 202) to begin the infusion process for the correct patient. According to various implementations, the APR message is sent to the coordination engine 212. Messages received by the coordination engine 212 may be stored in a tracing database. When the coordination engine determines that the order information is complete and that a system time associated with the EMR system 210 is synchronized with a system time associated with the coordination engine 212 (e.g., within ± 15 seconds), the APR message is accepted and an acknowledgement message may be sent back to the EMR system 210. In some implementations, an Infusion Administration Verification screen on the workstation of the caregiver displays a first confirmation at this stage in the process of the APR message. If there are incomplete fields or invalid data (e.g., wrong number of digits in the serial number field) or the system times are not synchronized, an error message may be sent to the EMR system 210, and an error message may be provided to the caregiver.

In some implementations, an infusion process begins when a medical personnel (e.g., a doctor) prescribes an infusion order for a medication to a patient. The same or a different medical personnel (e.g., a pharmacist, a nurse) may then verify the infusion order and the order is added to the patient's medical record in the EMR system 210. A caregiver (e.g., a nurse) at a site where the patient is to receive the infusion can access the medical record of the patient from the caregiver's workstation, or the infusion device, and may be notified of when a pending infusion order is to be provided to the patient. The caregiver may then retrieve a container of medication specified in the infusion order from a pharmacy or medication storage and prepares for the infusion. In some implementations, the bag identifier 234 may be affixed on the infusion container 230 by the caregiver administering the infusion process.

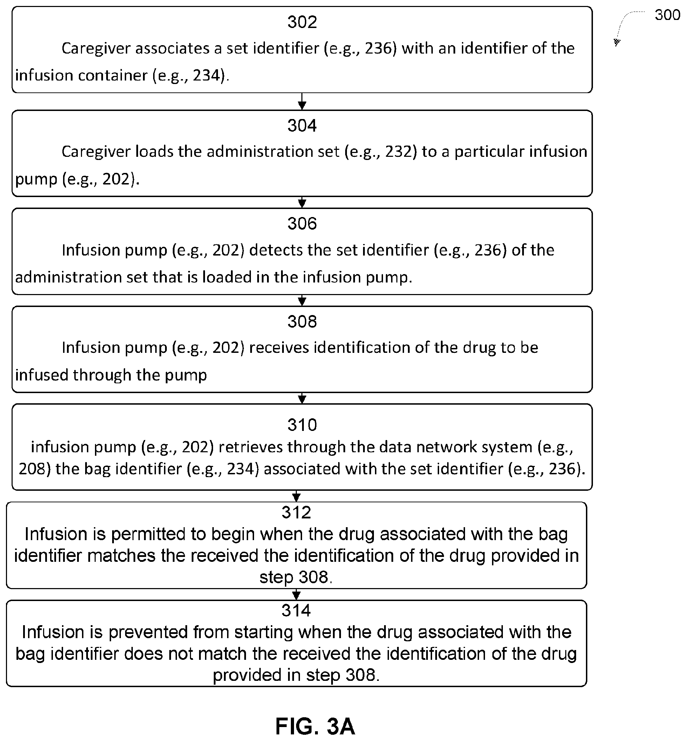

FIG. 3A depicts a first example workflow 300, according to aspects of the subject technology. A caregiver associates, at a step 302, a set identifier (e.g., 236) with an identifier of the infusion container (e.g., 234). In some implementations, associating the set identifier with the bag identifier involves sequentially scanning the set identifier 236 and the bag identifier 234.

In some implementations, a software installed or deployed on a caregiver's workstation, or within the infusion device 204, receives and processes communication data from the scanner 238 communicatively coupled to the workstation or the infusion device 204 (e.g., via Bluetooth, Bluetooth Low Energy, Wi-Fi, near-field communication (NFC), ZigBee, etc.). The scanner allows the caregiver to associate the bag identifier 234 to the set identifier 236. For example, the scanner 238 may be controlled by software operating on the infusion device 204, or on the caregiver's workstation to expect, in a first scan, the bag identifier. When the scanner transmits the information to the infusion device 204 or workstation, and the scanner may then be controlled to collect, via a second scan, the set identifier (e.g., 236). After the scanner transmits the information set identifier 236, the scanner may then be controlled to collect, via a third scan, a unique identifier associated with the infusion pump 202. In some implementations, the infusion device 204 processes the sequentially transmitted scanned bag identifier and the set identifier, pairs the data and sends the paired dataset to the data network system 208.

In some implementations, instead of sequentially transmitting the scanned bag identifier and set identifier to the infusion device 204, the set identifier and the bag identifier recorded by the scanner are transmitted as a paired dataset to the infusion device 204 or the data network system 208. The paired dataset records the pairing of the particular bag identifier to the particular set identifier. In this way, a search in a database of the data network system 208 for the bag identifier may also return the corresponding paired set identifier associated with the bag identifier.

As described earlier, the bag identifier may also provide identification of the drug that is housed within the infusion container. The drug information associated with the bag identifier may be provided to the central system by a different caregiver (e.g., a pharmacist) than the caregiver who is associating the bag identifier to the set identifier.

The caregiver, at a step 304, loads the administration set 232 to a particular infusion pump 202 (or module) in the infusion device 204. Loading the administration set 232 may involve mechanically engaging the administration set to a corresponding receiving receptacle in the pump 202.

The infusion pump 202 includes electronics and circuitry, and at a step 306, detects the set identifier of the administration set that is loaded in the infusion pump 202. In some implementations, the set identifier 236 may emit a wireless signal that is detected by the pump 202. In some implementations, the set identifier 236 may send an electrical signal to the infusion pump 202, for example, through an electrical connection mediated through the receiving receptacle in the pump 202.

The infusion pump 202 receives, in a step 308, identification of the drug to be infused through the pump 202. In some implementations, the drug identification is received from the data network system 208. For example, the drug identification is extracted from an APR sent to the infusion device 204 from the data network system 208. In some implementations, the caregiver provides the drug identification at the infusion device 204, for example, using a touch screen or softkeys at the infusion device 204 to select the drug to be infused.

Once the set identifier 236 is obtained in Step 306, the infusion pump 202 can retrieve, at a step 310, through the data network system 208, the bag identifier 234 associated with the set identifier 236. In some implementations, the data network system 208, retrieves the drug information associated with the bag identifier 234, through the pairing association, and sends the drug information to the infusion device 204.

The infusion device 204 evaluates (e.g., compares) the drug information received through the pairing association, with the drug information obtained from the step 308. When the infusion device 204 determines that the drug information matches, the infusion device 204, at a step 312, activates or lights a "start infusion" button on the infusion pump 202, allowing the caregiver to trigger the "start infusion" button to begin the infusion process.

In some implementations, the drug information provided by the caregiver in the step 308 is transmitted, via the communication module 206 to the data network system 208. The data network system 208 may then evaluate (e.g., compare) the received drug information with the drug information from the pairing association. When the data network system 208 determines that the drug information matches, the data network system 208 sends a control signal to the infusion device 204 permitting the infusion process to begin at the infusion pump 202. For example, the control signal allows the infusion process to begin (e.g., by presenting, activating or lighting a control element, such as a "start infusion" button on a display of the infusion pump 202) once the caregiver triggers the "start" button to activate the pump for delivering the content of the infusion container. In some implementations, the infusion device 204 interprets the data associated with the infusion order as a series of manual button presses. In some implementations, the infusion device 204 also checks that the medication contained in the infusion order exists in the online drug library 214 and that the dosing parameters are correct/valid.

If the drug associated with bag identifier does not correspond to the received drug identification provided in the step 308, the infusion pump 202 may, at a step 314, prevent the infusion process from starting (e.g., by deactivating a trigger button on the infusion pump 202 that allows the infusion process to start). In some implementations, the infusion device may prompt the caregiver to take additional remedial actions. For example, the infusion device 204 may generate an alert, or ay provide an interface update on a display of the infusion device, and or transmit messages to various systems within the data network system 208.

The association process reduces line error by automatically checking the accuracy of the association of the bag, the administration set and the infusion pump after the caregiver scans the various identifiers. The infusion system 200 is also able to monitor a usage metric for the administration set 232. For example, a processor within the infusion system 200 (e.g., within the infusion device 204, or external to the infusion device 204) is configured to generate a usage metric for the administration set based at least in part on a time when the pump starts infusing the content of the infusion container. The processor is configured to detect non-compliance of the usage metric with a safety threshold; and cause an adjustment to at least one physical characteristic of the infusion system. For example, the physical characteristic may include a sound alarm to alert a caregiver that the administration set 232 has been used for a time period exceeding a safety threshold (e.g., 24 hours, or 48 hours). The physical characteristic may also include a visual alarm presented on a display of the infusion device 204.

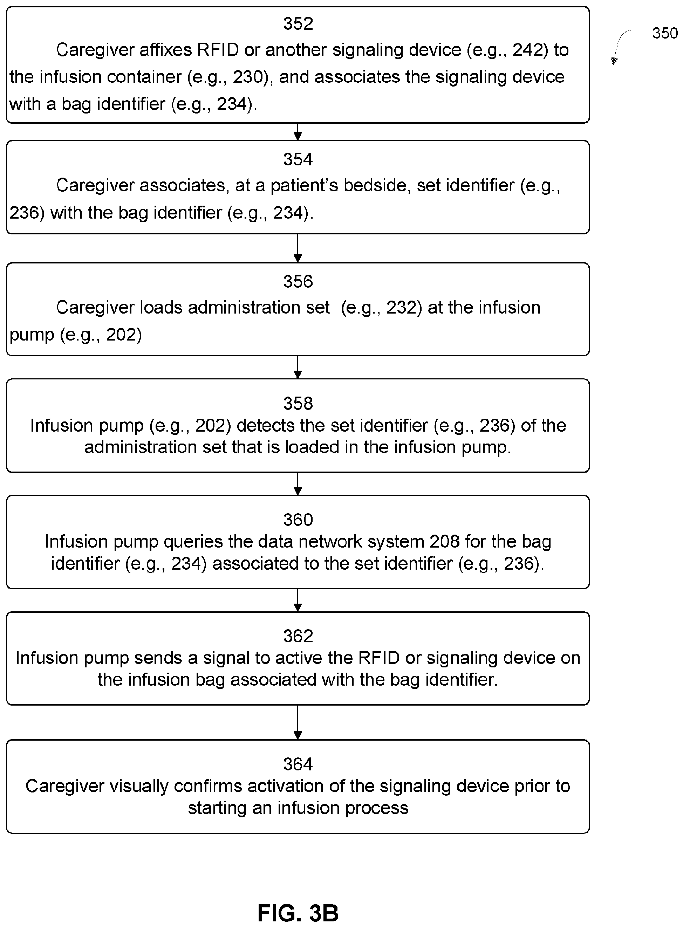

FIG. 3B shows a second example workflow 350, according to aspects of the subject technology. A caregiver, at a step 352, affixes an RFID or other signaling device (e.g., 242) to the infusion container (e.g., 230), and the RFID is associated with a bag identifier (e.g., 234) of the infusion container (e.g., 230). Associating the RFID or signaling device to the infusion container 230 may include scanning, by the caregiver, the RFID or signaling device, and the bag identifier, sequentially. In some implementations, the scanner may be controlled by software operating on the caregiver's workstation to expect, an RF signal from the RFID. When the scanner transmits the information to the workstation, the scanner may then be controlled to collect, via a scan, the bag identifier 234. A processor processes the collected data and pairs the signaling device 242 to the bag identifier 234. The processor may be a processor in the caregiver's workstation, or the processor may be a local processor on the scanner 238, which then causes the paired dataset to be transmitted to the caregiver's workstation. The paired dataset creates a first association between the bag identifier and RFID and the signaling device. In some implementations, only the association information (not the raw scanned data) is transmitted to the data network system 208.

At the site where the infusion is to be carried out (e.g., a patient's bedside), the set identifier (e.g., 236) is associated, in a step 354, with the bag identifier. Associating the set identifier with the bag identifier may use the process described with respect to the first example workflow 300. For example, a caregiver administering the infusion to the patient (e.g., a nurse, a nurse assistant, a doctor) may use a scanner that is controlled by software operating on the infusion device 204, or on the caregiver's workstation to expect, in a first scan, the bag identifier. When the scanner transmits the information to the infusion device or workstation, the central system may ascertain that the bag identifier has already been associated in the system to the RFID, by the caregiver. The scanner may then be controlled to collect, via a second scan, the set identifier. The association data for the bag identifier and the set identifier may be processed by the infusion device 204 or the workstation, and be sent to the data network system 208. Alternatively, the collected data may be sent directly to the data network system 208, and the association data is extracted and processed (e.g., paired) by a processor at the data network system 208.

The administration set is loaded, at a step 356, into the infusion pump (e.g., 202). Loading the administration set may involve mechanically engaging the administration set to a corresponding receiving receptacle in the pump 202.

The infusion pump 202 includes electronics and circuitry and detects, at a step 356, the set identifier (e.g., 236) at the infusion pump 202. In some implementations, the set identifier 236 may emit a wireless signal that is detected by the infusion pump 202. In some implementations, the set identifier 236 may send an electrical signal to the pump 202, for example, through an electrical connection mediated through the receiving receptacle in the infusion pump 202.

Based on the set identifier detected in the step 358, the infusion device 204 queries, at a step 360, a paired associational information database of the data network system 208 for the bag identifier 234 that is matched to the set identifier 236.

Based on the bag identifier determined at the step 360, the infusion device queries, at a step 362, the paired associational information database of the data network system 208, for the identifier of the RFID or signaling device 242 that is affixed to the infusion container 230.

The infusion device 204 sends, at a step 362, a signal to activate the RFID or signaling device 242 identified in the step 360. In some implementations, a wireless signal may be transmitted by a component, other than the infusion device 204, communicatively coupled to the data network system 208 to activate the RFID or signaling device 242.

The signal used to activate the RFID or the signaling device may cause a visual change. For example, an LED near or on the RFID or signaling device 242 may be lit upon the RFID or signaling device receiving the activation signal. The caregiver visually confirms, at a step 364, activation of the signaling device 242 prior to starting an infusion process.

In some implementations, the associational information is processed locally at the infusion device 204 or the caregiver's workstation without relying on the data network system 208. In some implementations, the associational information scanned by the caregiver is relayed to and displayed on the caregiver's workstation and/or the infusion device. In some implementations, the second example workflow 350 allows the caregiver to ensure that a particular medical fluid prepared by a different caregiver at a first location (e.g., the pharmacy) is correctly prepared for infusion to a particular patient at a second location (e.g., bedside). In addition, the correct administration set and the correct infusion pump are correctly associated and recorded in the data network system 208.

In some implementations, the signal in the step 362 used to activate the RFID or the signaling device 242 activates an LED of a particular color, and the infusion pump 202 to which the administration set is loaded also has an indicator light of the same color. This may further assist the caregiver to more quickly confirm the correction association of the infusion container at a particular infusion pump, without having to conduct time-consuming physical infusion line tracing.

In some implementations, the administration set may further include a fiber optic thread (e.g., on or within the tubing). An additional light emitter may also be provided in the infusion pump. The light emitter may direct light along the fiber optic thread, allowing a caregiver to visually confirm the correct association of the administration set to both the infusion pump and the infusion container.

In some implementations, an infusion order is sent down to the infusion device 204 from a data network system 208 (e.g., the EMR 210). The infusion device 204 may control up to four infusion pumps (channels).

The caregiver is able to decide the actual sequence of scanning. In some implementations, the caregiver loads the administration set (having an attached infusion container) into the infusion pump before scanning the bag identifier and the set identifier. The data network system 208 may decide, based on the received association data, which infusion pump of the infusion device to send the infusion order to. In some implementations, the infusion device detects which administration set was most recently loaded, and the infusion order received from the central system to the infusion device is routed to the infusion pump having the most recently loaded administration set.

Scanning the set identifier also allows the data network system 208 and/or the infusion device 204 to keep track of how long the set identifier has been loaded into the infusion pump and how long the administration set has been put in use.

In some implementations, the infusion device may hold multiple APRs. Once the administration set is loaded into the particular infusion pump, the infusion device is able to determine, from the set identifier (and the associated bag identifier containing the medical fluid) which of the multiple APRs contains the infusion order that corresponds to the set identifier. In some implementations, the display at the infusion device may show that the first order is being initiated at the infusion pump. In some implementations, an indicator light associated with the infusion pump may also light up.

According to various implementations, the systems and methods cross checks the infusion container and the administration set association, and ensure that the identifiers (e.g., bag identifier and set identifier) that are scanned are loaded in a pumping channel that correctly receives the infusion order from the data network system 208. The infusion device 204 may have the hardware to detect the set identifier of the ID set (e.g., ID1). In addition, the infusion device 204 (e.g., PCU) also receives a set identifier (e.g., ID2) that was scanned and sent wirelessly. The infusion device then checks that the set identifier detected at the infusion pump matches the set identifier that was received wirelessly (e.g., ID 1 matches ID2).

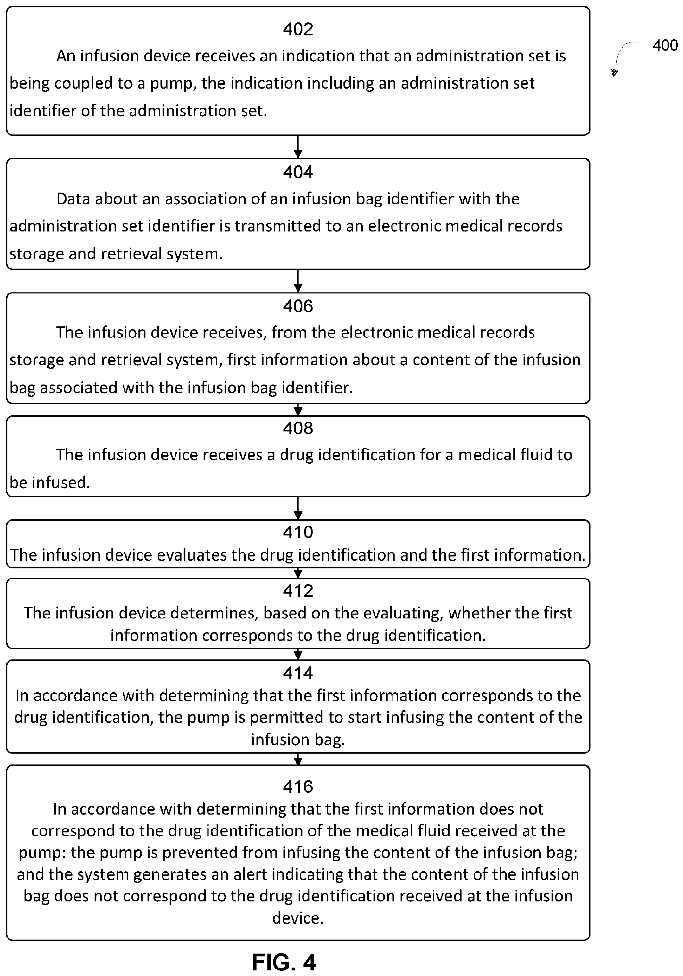

FIG. 4 depicts an example method for associating an infusion container, an administration set, and a pump of an infusion device, according to aspects of the subject technology. For explanatory purposes, the various blocks of example method 400 are described herein with reference to FIGS. 1-3, and the components and/or processes described herein. The one or more of the blocks of method 400 may be implemented, for example, by one or more computing devices such as those described above. In some implementations, one or more of the blocks may be implemented apart from other blocks, and by one or more different processors or devices. Further for explanatory purposes, the blocks of example method 400 are described as occurring in serial, or linearly. However, multiple blocks of example method 400 may occur in parallel. In addition, the blocks of example method 400 need not be performed in the order shown and/or one or more of the blocks of example method 400 need not be performed.

In the depicted example, an infusion device receives an indication that the administration set is being coupled to the pump, the indication including an administration set identifier of the administration set (402). The infusion device transmits to an electronic medical records storage and retrieval system, data about an association of an infusion container identifier with the administration set identifier (404). The infusion device receives from the electronic medical records storage and retrieval system, first information about a content of the infusion container associated with the infusion container identifier (406). The infusion device receives a drug identification for a medical fluid to be infused (408). The infusion device evaluates (e.g., compares) the drug identification with the first information about the content of the infusion container (410). For example, a drug name and drug concentration are extracted from the first information about the content of the infusion container, and compared against the drug name and drug concentration provided in the drug identification. The infusion device determines, based on the evaluating, whether the first information corresponds to the drug identification (412). In accordance with a determination that the first information about the content of the infusion container corresponds to (e.g., matches) the drug identification: the pump is permitted to start infusing the content of the infusion container (414). For example, the drug name from the first information about the content of the infusion container, matches the drug name provided in the drug identification. In accordance with a determination that the first information about the content of the infusion container does not match the drug identification of the medical fluid received at the pump: the pump is prevented from infusing the content of the infusion container; and the system generates an alert indicating that the content of the infusion container does not match the drug identification received at the infusion device (416).

In some implementations, an infusion system includes an administration set receiver; a pump disposed to provide a force to at least a portion of an administration set after the administration set is received by the administration set receiver; a network data transceiver; and a processor configured to: detect an administration set identifier for the administration set received by the administration set receiver; transmit, via the network data transceiver to an electronic medical records storage and retrieval system, a message identifying an association between an infusion container identifier and the administration set identifier; receive, via the network data transceiver from the electronic medical records storage and retrieval system, first information about a content of the infusion container associated with the infusion container identifier; receive a drug identification for a medical fluid to be infused; determine that the first information corresponds to the drug identification; and permit the pump to start infusing the content of the infusion container.

In some implementations, the administration set receiver includes: an administration set key channel for receiving an administration set key included on the administration set; and a sensor having a detection field of at least a portion of the administration set key channel; and detecting the administration set identifier includes: activating the sensor; and receiving a value from the sensor detected from the administration set key received by the administration key channel.

In some implementations, the processor is configured to receive the infusion container identifier from an optical scanning device. In some implementations, the infusion system further includes a wireless scanning device having a scanning range covering from the pump to the infusion container. The processor is configured to receive the infusion container identifier from the wireless scanning device after the infusion container enters the scanning range. In some implementations, permitting the pump to start infusing includes presenting, via a display of the infusion system, a control element to activate the pump for delivering the content of the infusion container. In some implementations, the processor is configured to: generate a usage metric for the administration set based at least in part on a time when the pump starts infusing the content. The processor is configured to: detect non-compliance of the usage metric with a safety threshold; and cause an adjustment to at least one physical characteristic of the infusion system.

In some implementations, a method of associating an infusion container, an administration set, and a pump of an infusion device includes receiving, at an infusion device, an indication that an administration set is being coupled to a pump, the indication including an administration set identifier of the administration set. The method includes transmitting to an electronic medical records storage and retrieval system, data about an association of an infusion container identifier with the administration set identifier. The method includes receiving, from the electronic medical records storage and retrieval system, first information about a content of the infusion container associated with the infusion container identifier. The method includes receiving, at the infusion device, a drug identification for a medical fluid to be infused. The method includes evaluating, at the infusion device, the drug identification with the first information about the content of the infusion container. The method includes determining, based on the evaluating, whether the first information corresponds to the drug identification. In accordance with determining that the first information about the content of the infusion container matches the drug identification: permitting the pump to start infusing the content of the infusion container. And in accordance with determining that the first information about the content of the infusion container does not match the drug identification of the medical fluid received at the pump: preventing the pump from infusing the content of the infusion container; and generating an alert indicating that the content of the infusion container does not match the drug identification received at the infusion device.

In some implementations, the infusion container identifier includes a first 2D barcode, the administration set identifier includes a second 2D barcode. The data about the association of the infusion container identifier with the administration set identifier includes data obtained from sequentially scanning the first 2D barcode and the second 2D barcode. In some implementations, scanner used for scanning the first 2D barcode and the second 2D barcode includes a communication unit that transmits scanned information to the infusion device. In some implementations, electronic medical records storage and retrieval system receives and stores the association of an infusion container identifier with the administration set identifier. In some implementations, the method further includes monitoring, by the infusion device, a usage metric of the administration set after the administration set is coupled with the pump. In some implementations, receiving, at the infusion device, the drug identification for the medical fluid to be infused includes receiving manually entered data. In some implementations, receiving, at the infusion device, the drug identification for the medical fluid to be infused includes receiving the drug identification from an infusion order sent to the infusion device from the electronic medical records storage and retrieval system. In some implementations, the infusion device is configured to store a plurality of infusion orders, and automatically downloading infusion parameters associated with a respective one of the plurality of infusion orders involving the medical fluid to the pump the administration set is coupled to. In some implementations, transmitting the data about the association of the infusion container identifier and the administration set identifier occurs after the administration set is coupled to the pump. In some implementations, the electronic medical records storage and retrieval system sends infusion order information to the pump the administration set is coupled to, after determining that the first information about the content of the infusion container matches the drug identification of the medical fluid. In some implementations, the data about the association of the infusion container identifier and the administration set identifier and are generated before the administration set is coupled to the pump. In some implementations, a method of infusing the medical fluid to a patient, includes associating the infusion container, the administration set, and the pump of the infusion device prior to enabling the infusion pump to begin infusing the content of the infusion container using a one-touch start button at the infusion device.

In some implementations, a method of associating an infusion container, an administration set, and a pump of an infusion device, the method includes receiving an indication that a signaling device has been affixed to an infusion container, and receiving data associating the signaling device with an infusion container identifier. The method includes receiving data associating an administration set identifier with infusion container identifier. The method includes transmitting, to an electronic medical records storage and retrieval system, information associating the infusion container identifier and the administration set identifier. The method includes receiving an indication that the administration set is being coupled to the pump, the indication including the administration set identifier. The method includes retrieving, from the electronic medical records storage and retrieval system, the infusion container identifier based on the administration set identifier. The method includes retrieving, from the electronic medical records storage and retrieval system, the data associating the signaling device with the infusion container, based on infusion container identifier. The method includes transmitting, by the infusion device, a signal to the signaling device to activate a visual display indicating that the pump is associated with the infusion container.

In some implementations, the electronic medical records storage and retrieval system includes data about a content of the infusion container associated with the identifier of the infusion container. In some implementations, the signaling device includes a RFID and a plurality of LED emitting light of different colors. In some implementations the signaling device is affixed is different from a location where the administration set identifier and the infusion container identifier are associated. In some implementations, a signaling device is affixed at a pharmacy and the administration set identifier and the infusion container identifier are associated at a bedside of a patient.

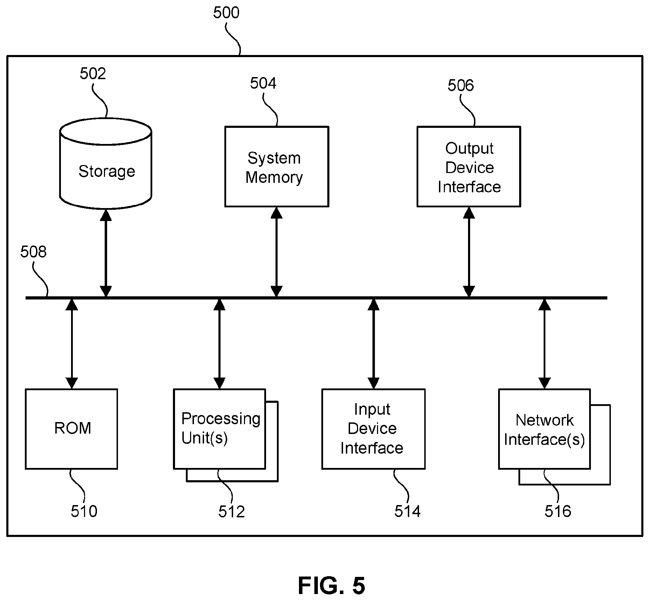

FIG. 5 is a conceptual diagram illustrating an example electronic system for associating an infusion container, an administration set, and a pump of an infusion device, according to aspects of the subject technology. Electronic system 500 may be a computing device for execution of software associated with one or more portions or steps of process, or components and processes provided by FIGS. 1-3, including but not limited to server 130, computing hardware within patient care device 12, or terminal device 132. Electronic system 500 may be representative, in combination with the disclosure regarding FIGS. 1-3. In this regard, electronic system 500 may be a personal computer or a mobile device such as a smartphone, tablet computer, laptop, PDA, an augmented reality device, a wearable such as a watch or band or glasses, or combination thereof, or other touch screen or television with one or more processors embedded therein or coupled thereto, or any other sort of computer-related electronic device having network connectivity.

Electronic system 500 may include various types of computer readable media and interfaces for various other types of computer readable media. In the depicted example, electronic system 500 includes a bus 508, processing unit(s) 512, a system memory 504, a read-only memory (ROM) 510, a permanent storage device 502, an input device interface 514, an output device interface 506, and one or more network interfaces 516. In some implementations, electronic system 500 may include or be integrated with other computing devices or circuitry for operation of the various components and processes previously described.

Bus 508 collectively represents all system, peripheral, and chipset buses that communicatively connect the numerous internal devices of electronic system 500. For instance, bus 508 communicatively connects processing unit(s) 512 with ROM 510, system memory 504, and permanent storage device 502.

From these various memory units, processing unit(s) 512 retrieves instructions to execute and data to process in order to execute the processes of the subject disclosure. The processing unit(s) can be a single processor or a multi-core processor in different implementations.

ROM 510 stores static data and instructions that are needed by processing unit(s) 512 and other modules of the electronic system. Permanent storage device 502, on the other hand, is a read-and-write memory device. This device is a non-volatile memory unit that stores instructions and data even when electronic system 500 is off. Some implementations of the subject disclosure use a mass-storage device (such as a magnetic or optical disk and its corresponding disk drive) as permanent storage device 502.

Other implementations use a removable storage device (such as a floppy disk, flash drive, and its corresponding disk drive) as permanent storage device 502. Like permanent storage device 502, system memory 504 is a read-and-write memory device. However, unlike storage device 502, system memory 504 is a volatile read-and-write memory, such a random access memory. System memory 504 stores some of the instructions and data that the processor needs at runtime. In some implementations, the processes of the subject disclosure are stored in system memory 504, permanent storage device 502, and/or ROM 510. From these various memory units, processing unit(s) 512 retrieves instructions to execute and data to process in order to execute the processes of some implementations.

Bus 508 also connects to input and output device interfaces 514 and 506. Input device interface 514 enables the user to communicate information and select commands to the electronic system. Input devices used with input device interface 514 include, e.g., alphanumeric keyboards and pointing devices (also called "cursor control devices"). Output device interfaces 506 enables, e.g., the display of images generated by the electronic system 500. Output devices used with output device interface 506 include, e.g., printers and display devices, such as cathode ray tubes (CRT) or liquid crystal displays (LCD). Some implementations include devices such as a touchscreen that functions as both input and output devices.

Also, as shown in FIG. 5, bus 508 also couples electronic system 500 to a network (not shown) through network interfaces 516. Network interfaces 516 may include, e.g., a wireless access point (e.g., Bluetooth or WiFi) or radio circuitry (e.g., transceiver, antenna, amplifier) for connecting to a wireless access point. Network interfaces 516 may also include hardware (e.g., Ethernet hardware) for connecting the computer to a part of a network of computers such as a local area network ("LAN"), a wide area network ("WAN"), wireless LAN, personal area network ("PAN"), or an Intranet, or a network of networks, such as the Internet. Any or all components of electronic system 500 can be used in conjunction with the subject disclosure.

These functions described above can be implemented in computer software, firmware or hardware. The techniques can be implemented using one or more computer program products. Programmable processors and computers can be included in or packaged as mobile devices. The processes and logic flows can be performed by one or more programmable processors and by one or more programmable logic circuitry. General and special purpose computing devices and storage devices specifically configured for the infusion features described can be interconnected through communication networks.

Some implementations include electronic components, such as microprocessors, storage and memory that store computer program instructions in a machine-readable or computer-readable medium (also referred to as computer-readable storage media, machine-readable media, or machine-readable storage media). Some examples of such computer-readable media include RAM, ROM, read-only compact discs (CD-ROM), recordable compact discs (CD-R), rewritable compact discs (CD-RW), read-only digital versatile discs (e.g., DVD-ROM, dual-layer DVD-ROM), a variety of recordable/rewritable DVDs (e.g., DVD-RAM, DVD-RW, DVD+RW, etc.), flash memory (e.g., SD cards, mini-SD cards, micro-SD cards, etc.), magnetic and/or solid state hard drives, read-only and recordable Blu-Ray^{®} discs, ultra density optical discs, any other optical or magnetic media, and floppy disks. The computer-readable media can store a computer program that is executable by at least one processing unit and includes sets of instructions for performing various operations. Examples of computer programs or computer code include machine code, such as is produced by a compiler, and files including higher-level code that are executed by a computer, an electronic component, or a microprocessor using an interpreter.

While the above discussion primarily refers to microprocessor or multi-core processors that execute software, some implementations are performed by one or more integrated circuits, such as application specific integrated circuits (ASICs) or field programmable gate arrays (FPGAs). In some implementations, such integrated circuits execute instructions that are stored on the circuit itself.

As used in this specification and any claims of this application, the terms "computer", "server", "processor", and "memory" all refer to electronic or other technological devices. These terms exclude people or groups of people. For the purposes of the specification, the terms display or displaying means displaying on an electronic device. As used in this specification and any claims of this application, the terms "computer readable medium" and "computer readable media" are entirely restricted to tangible, physical objects that store information in a form that is readable by a computer. These terms exclude any wireless signals, wired download signals, and any other ephemeral signals.

To provide for interaction with a user, implementations of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; e.g., feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; e.g., by sending web pages to a web browser on a user's client device in response to requests received from the web browser.

Embodiments of the subject matter described in this specification can be implemented in a specifically configured computing system that includes a back end component, e.g., as a data server, or that includes a middleware component, e.g., an application server, or that includes a front end component, e.g., a client computer having a graphical user interface or a Web browser through which a user can interact with an implementation of the subject matter described in this specification, or any combination of one or more such back end, middleware, or front end components. The components of the system can be interconnected by any form or medium of digital data communication, e.g., a communication network. Examples of communication networks include a local area network ("LAN") and a wide area network ("WAN"), an internetwork (e.g., the Internet), and peer-to-peer networks (e.g., ad hoc peer-to-peer networks).

The computing system can include clients and servers. A client and server are generally remote from each other and may interact through a communication network. The relationship of client and server arises by virtue of computer programs running on the respective computers and having a client-server relationship to each other. In some implementations, a server transmits data (e.g., an HTML page) to a client device (e.g., for purposes of displaying data to and receiving user input from a user interacting with the client device). Data generated at the client device (e.g., a result of the user interaction) can be received from the client device at the server.

Those of skill in the art would appreciate that the various illustrative blocks, modules, elements, components, methods, and algorithms described herein may be implemented as electronic hardware, computer software, or combinations of both. To illustrate this interchangeability of hardware and software, various illustrative blocks, modules, elements, components, methods, and algorithms have been described above generally in terms of their functionality. Whether such functionality is implemented as hardware or software depends upon the particular application and design constraints imposed on the overall system. The described functionality may be implemented in varying ways for each particular application. Various components and blocks may be arranged differently (e.g., arranged in a different order, or partitioned in a different way) all without departing from the scope of the subject technology.

It is understood that the specific order or hierarchy of steps in the processes disclosed is an illustration of example approaches. Based upon design preferences, it is understood that the specific order or hierarchy of steps in the processes may be rearranged. Some of the steps may be performed simultaneously.

The previous description is provided to enable any person skilled in the art to practice the various aspects described herein. The previous description provides various examples of the subject technology, and the subject technology is not limited to these examples. Various modifications to these aspects will be readily apparent to those skilled in the art, and the generic principles defined herein may be applied to other aspects. Thus, the claims are not intended to be limited to the aspects shown herein, but is to be accorded the full scope consistent with the language claims, wherein reference to an element in the singular is not intended to mean "one and only one" unless specifically so stated, but rather "one or more." Unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the invention described herein.

The term website, as used herein, may include any aspect of a website, including one or more web pages, one or more servers used to host or store web related content, etc. Accordingly, the term website may be used interchangeably with the terms web page and server. The predicate words "configured to", "operable to", and "programmed to" do not imply any particular tangible or intangible modification of a subject, but, rather, are intended to be used interchangeably. For example, a processor configured to monitor and control an operation or a component may also mean the processor being programmed to monitor and control the operation or the processor being operable to monitor and control the operation. Likewise, a processor configured to execute code can be construed as a processor programmed to execute code or operable to execute code.

The term automatic, as used herein, may include performance by a computer or machine without user intervention; for example, by instructions responsive to a predicate action by the computer or machine or other initiation mechanism. The word "example" is used herein to mean "serving as an example or illustration." Any aspect or design described herein as "example" is not necessarily to be construed as preferred or advantageous over other aspects or designs.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "embodiment" does not imply that such embodiment is essential to the subject technology or that such embodiment applies to all configurations of the subject technology. A disclosure relating to an embodiment may apply to all implementations, or one or more implementations. An embodiment may provide one or more examples. A phrase such as an "embodiment" may refer to one or more embodiments and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such as a "configuration" may refer to one or more configurations and vice versa.

As used herein, the terms "determine" or "determining" encompass a wide variety of actions. For example, "determining" may include calculating, computing, processing, deriving, generating, obtaining, looking up (e.g., looking up in a table, a database or another data structure), ascertaining and the like via a hardware element without user intervention. Also, "determining" may include receiving (e.g., receiving information), accessing (e.g., accessing data in a memory) and the like via a hardware element without user intervention. "Determining" may include resolving, selecting, choosing, establishing, and the like via a hardware element without user intervention.

As used herein, the terms "provide" or "providing" encompass a wide variety of actions. For example, "providing" may include storing a value in a location of a storage device for subsequent retrieval, transmitting a value directly to the recipient via at least one wired or wireless communication medium, transmitting or storing a reference to a value, and the like. "Providing" may also include encoding, decoding, encrypting, decrypting, validating, verifying, and the like via a hardware element.

As used herein, the term "message" encompasses a wide variety of formats for communicating (e.g., transmitting or receiving) information. A message may include a machine readable aggregation of information such as an XML document, fixed field message, comma separated message, JSON, a custom protocol, or the like. A message may, in some embodiments, include a signal utilized to transmit one or more representations of the information. While recited in the singular, it will be understood that a message may be composed, transmitted, stored, received, etc. in multiple parts.

As used herein, the terms "correspond" or "corresponding" encompasses a structural, functional, quantitative and/or qualitative correlation or relationship between two or more objects, data sets, information and/or the like, preferably where the correspondence or relationship may be used to translate one or more of the two or more objects, data sets, information and/or the like so to appear to be the same or equal. Correspondence may be assessed using one or more of a threshold, a value range, fuzzy logic, pattern matching, a machine learning assessment model, or combinations thereof.

In any embodiment, data generated or detected can be forwarded to a "remote" device or location, where "remote," means a location or device other than the location or device at which the program is executed. For example, a remote location could be another location (e.g., office, lab, etc.) in the same city, another location in a different city, another location in a different state, another location in a different country, etc. As such, when one item is indicated as being "remote" from another, what is meant is that the two items can be in the same room but separated, or at least in different rooms or different buildings, and can be at least one mile, ten miles, or at least one hundred miles apart. "Communicating" information references transmitting the data representing that information as electrical signals over a suitable communication channel (e.g., a private or public network). "Forwarding" an item refers to any means of getting that item from one location to the next, whether by physically transporting that item or otherwise (where that is possible) and includes, at least in the case of data, physically transporting a medium carrying the data or communicating the data. Examples of communicating media include radio or infra-red transmission channels as well as a network connection to another computer or networked device, and the internet or cellular networks.

As used herein a "user interface" (also referred to as an interactive user interface, a graphical user interface or a UI) may refer to a network based interface including data fields and/or other control elements for receiving input signals or providing electronic information and/or for providing information to the user in response to any received input signals. Control elements may include dials, buttons, icons, selectable areas, or other perceivable indicia presented via the UI that, when interacted with (e.g., clicked, touched, selected, etc.), initiates an exchange of data for the device presenting the UI. A UI may be implemented in whole or in part using technologies such as hyper-text mark-up language (HTML), FLASH^{™}, JAVA^{™}, .NET^{™}, web services, or rich site summary (RSS). In some implementations, a UI may be included in a stand-alone client (for example, thick client, fat client) configured to communicate (e.g., send or receive data) in accordance with one or more of the aspects described. The communication may be to or from a medical device, diagnostic device, monitoring device, or server in communication therewith.

## Claims

1. An infusion system (200) comprising:
an administration set receiver (246);
a pump (202) disposed to provide a force to at least a portion of a tube of an administration set after the administration set is received by the administration set receiver to pump a fluid through the administration set;
a network data transceiver (206); and
a processor configured to:
detect (306), by the administration set receiver, an administration set identifier for the administration set transmitted by a communication module included in the administration set when the administration set is loaded in the pump;
transmit (310), via the network data transceiver to an electronic medical records storage and retrieval system, the administration set identifier;
receive (310), via the network data transceiver from the electronic medical records storage and retrieval system, drug information about a content of an infusion container associated with the administration set identifier;
receive (308), at the pump, separately from the received drug information, a drug identification for a medical fluid to be infused by the pump;
determine whether the received drug information corresponds to the received drug identification; and
permit (312) the pump to start an infusion of the medical fluid when the received drug information corresponds to the received drug identification, and
prevent (314) the pump from starting the infusion when the received drug information does not correspond to the received drug identification.

2. The infusion system of claim 1, wherein detecting the administration set identifier includes:
activating a sensor; and
receiving a value from the sensor detected from the administration set.

3. The infusion system of claim 1 or 2, wherein the processor being configured to receive the drug identification comprises the processor being configured to extract the drug identification from an auto programming request message sent to the pump from a data network system.

4. The infusion system of claim 3, wherein the pump receives multiple auto programming request messages, and
wherein the processor is configured to:
receive an infusion order from the electronic medical records storage and retrieval system; and
determine, from the administration set identifier, which of the multiple auto programming request messages contains an infusion order that corresponds to the set identifier.

5. The infusion system of any one of claims 1 to 4, wherein permitting the pump to start infusing includes presenting, via a display of the infusion system, a control element to activate the pump for delivering the content of the infusion container.

6. The infusion system of any of claims 1 to 5, wherein the processor is configured to:
generate a usage metric for the administration set based at least in part on a time when the pump starts infusing the content.

7. The infusion system of claim 6, wherein the processor is configured to:
detect non-compliance of the usage metric with a safety threshold; and
sound an alarm to alert a caregiver when the usage metric indicates that the administration set has been used for a time period exceeding a safety threshold.

8. A non-transitory machine readable medium embodying instructions that, when executed by a data processing machine, cause the machine to perform a method of associating an infusion container, an administration set, and a pump of an infusion device, the method comprising:
receiving, at an infusion device, an indication that an administration set is being coupled to a pump, the administration set comprising a tubing to which a force is applied by the pump to pump a fluid through the administration set;
detecting, by an administration set receiver of the infusion device, an administration set identifier of the administration set, the administration set identifier being transmitted by a communication module included in the administration set when the administration set is coupled to the pump;
transmitting to an electronic medical records storage and retrieval system, the administration set identifier;
receiving, from the electronic medical records storage and retrieval system, drug information about a content of an infusion container associated with the administration set identifier;
receiving, at the infusion device, separately from the received drug information, a drug identification for a medical fluid to be infused;
evaluating, at the infusion device, the drug identification with the drug information;
determining, based on the evaluating, whether the drug information corresponds to the drug identification;
in accordance with determining that the drug information corresponds to the drug identification, permitting the pump to start an infusion of the medical fluid; and
in accordance with determining that the drug information does not correspond to the drug identification of the medical fluid received at the pump:
preventing the pump from starting the infusion; and
generating an alert indicating that an infusion container for the medical fluid does not correspond to the drug identification received at the infusion device.

9. The non-transitory machine readable medium of claim 8, wherein the electronic medical records storage and retrieval system receives and stores the association of an infusion container identifier with the administration set identifier.

10. The non-transitory machine readable medium of claim 8 or claim 9, the method further comprising monitoring, by the infusion device, a usage metric of the administration set after the administration set is coupled with the pump.

11. The non-transitory machine readable medium of any one of claims 8 to 10, wherein receiving, at the infusion device, the drug identification comprises receiving manually entered data.

12. The non-transitory machine readable medium of any one of claims 8 to 11, wherein receiving, at the infusion device, the drug identification comprises receiving the drug identification from an infusion order sent to the infusion device from the electronic medical records storage and retrieval system.

13. The non-transitory machine readable medium of any one of claims 8 to 12, wherein the infusion device is configured to store a plurality of infusion orders, and automatically downloading infusion parameters associated with a respective one of the plurality of infusion orders involving the medical fluid to the pump the administration set is coupled to.

14. The non-transitory machine readable medium of any one of claims 8 to 13, wherein transmitting the administration set identifier occurs after the administration set is coupled to the pump, and the electronic medical records storage and retrieval system sends infusion order information to the pump that the administration set is coupled to, after determining that the drug information matches the drug identification.

## Patentansprüche

1. Infusionssystem (200), umfassend: eine Infusionsset-Aufnahme (246); eine Pumpe (202), die dazu angeordnet ist, auf zumindest einen Teil eines Schlauchs eines Infusionssets eine Kraft auszuüben, nachdem das Infusionsset von der Infusionsset-Aufnahme aufgenommen wurde, um ein Fluid durch das Infusionsset zu pumpen; einen Netzwerk-Daten-Transceiver (206); und einen Prozessor, der dazu ausgebildet ist: durch die Infusionsset-Aufnahme eine Infusionsset-Kennung für das Infusionsset zu erkennen (306), die von einem in dem Infusionsset enthaltenen Kommunikationsmodul übermittelt wird, wenn das Infusionsset in die Pumpe geladen wird; über den Netzwerk-Daten-Transceiver an ein System zum Speichern und Abrufen elektronischer Krankenakten die Infusionsset-Kennung zu übermitteln (310); über den Netzwerk-Daten-Transceiver von dem System zum Speichern und Abrufen elektronischer Krankenakten Arzneimittelinformation über einen Inhalt eines mit der Infusionsset-Kennung verknüpften Infusionsbehälters zu empfangen (310); an der Pumpe, separat von der empfangenen Arzneimittelinformation, eine Arzneimittelidentifikation für ein von der Pumpe zu infundierendes medizinisches Fluid zu empfangen (308); zu bestimmen, ob die empfangene Arzneimittelinformation der empfangenen Arzneimittelidentifikation entspricht; und zuzulassen (312), dass die Pumpe eine Infusion des medizinischen Fluids startet, wenn die empfangene Arzneimittelinformation der empfangenen Arzneimittelidentifikation entspricht, und zu verhindern (314), dass die Pumpe die Infusion startet, wenn die empfangene Arzneimittelinformation der empfangenen Arzneimittelidentifikation nicht entspricht.

2. Infusionssystem nach Anspruch 1, wobei das Erkennen der Infusionsset-Kennung umfasst: Aktivieren eines Sensors; und Empfangen eines Werts von dem Sensor, wobei der Wert an dem Infusionsset detektiert wurde.

3. Infusionssystem nach Anspruch 1 oder 2, wobei das Ausgebildetsein des Prozessors zum Empfangen der Arzneimittelidentifikation umfasst, dass der Prozessor dazu ausgebildet ist, die Arzneimittelidentifikation aus einer automatischen Programmieranforderungs-Nachricht zu extrahieren, die von einem Datennetzwerksystem an die Pumpe gesendet wird.

4. Infusionssystem nach Anspruch 3, wobei die Pumpe mehrere automatische Programmieranforderungs-Nachrichten empfängt, und wobei der Prozessor dazu ausgebildet ist: einen Infusionsauftrag von dem System zum Speichern und Abrufen elektronischer Krankenakten zu empfangen; und aus der Infusionsset-Kennung zu bestimmen, welche der mehreren automatischen Programmieranforderungs-Nachrichten einen Infusionsauftrag enthält, der der Infusionsset-Kennung entspricht.

5. Infusionssystem nach einem der Ansprüche 1 bis 4, wobei das Zulassen, dass die Pumpe mit dem Infundieren beginnt, das Anzeigen eines Bedienelementes über eine Anzeige des Infusionssystems umfasst, um die Pumpe zur Abgabe des Inhalts des Infusionsbehälters zu aktivieren.

6. Infusionssystem nach einem der Ansprüche 1 bis 5, wobei der Prozessor dazu ausgebildet ist: eine Nutzungsmetrik für das Infusionsset zumindest teilweise basierend auf einem Zeitpunkt zu erzeugen, zu dem die Pumpe mit dem Infundieren des Inhalts beginnt.

7. Infusionssystem nach Anspruch 6, wobei der Prozessor dazu ausgebildet ist: eine Nichteinhaltung der Nutzungsmetrik gegenüber einem Sicherheitsschwellenwert zu erkennen; und einen Alarm auszulösen, um eine Pflegeperson zu warnen, wenn die Nutzungsmetrik anzeigt, dass das Infusionsset für einen Zeitraum verwendet wurde, der einen Sicherheitsschwellenwert überschreitet.

8. Nicht-flüchtiges maschinenlesbares Medium, das Anweisungen verkörpert, die, wenn sie von einer Datenverarbeitungsmaschine ausgeführt werden, die Maschine veranlassen, ein Verfahren zum Verknüpfen eines Infusionsbehälters, eines Infusionssets und einer Pumpe eines Infusionsgeräts durchzuführen, wobei das Verfahren umfasst:
Empfangen, an einem Infusionsgerät, einer Angabe, dass ein Infusionsset an eine Pumpe gekoppelt wird, wobei das Infusionsset einen Schlauch umfasst, auf den von der Pumpe eine Kraft ausgeübt wird, um ein Fluid durch das Infusionsset zu pumpen; Erkennen, durch eine Infusionsset-Aufnahme des Infusionsgeräts, einer Infusionsset-Kennung des Infusionssets, wobei die Infusionsset-Kennung von einem in dem Infusionsset enthaltenen Kommunikationsmodul übermittelt wird, wenn das Infusionsset an die Pumpe gekoppelt wird; Übermitteln der Infusionsset-Kennung an ein System zum Speichern und Abrufen elektronischer Krankenakten; Empfangen, von dem System zum Speichern und Abrufen elektronischer Krankenakten, von Arzneimittelinformation über einen Inhalt eines mit der Infusionsset-Kennung verknüpften Infusionsbehälters;
Empfangen, an dem Infusionsgerät, separat von der empfangenen Arzneimittelinformation, einer Arzneimittelidentifikation für ein zu infundierendes medizinisches Fluid; Auswerten, an dem Infusionsgerät, der Arzneimittelidentifikation im Vergleich mit der Arzneimittelinformation; Bestimmen, basierend auf dem Auswerten, ob die Arzneimittelinformation der Arzneimittelidentifikation entspricht; wenn festgestellt wird, dass die Arzneimittelinformation der Arzneimittelidentifikation entspricht, Zulassen, dass die Pumpe eine Infusion des medizinischen Fluids startet; und wenn festgestellt wird, dass die Arzneimittelinformation der Arzneimittelidentifikation für das an der Pumpe empfangene medizinische Fluid nicht entspricht: Verhindern, dass die Pumpe die Infusion startet; und Erzeugen einer Warnung, die anzeigt, dass ein Infusionsbehälter für das medizinische Fluid der an dem Infusionsgerät empfangenen Arzneimittelidentifikation nicht entspricht.

9. Nicht-flüchtiges maschinenlesbares Medium nach Anspruch 8, wobei das System zum Speichern und Abrufen elektronischer Krankenakten die Verknüpfung einer Infusionsbehälter-Kennung mit der Infusionsset-Kennung empfängt und speichert.

10. Nicht-flüchtiges maschinenlesbares Medium nach Anspruch 8 oder Anspruch 9, wobei das Verfahren ferner umfasst:
Überwachen, durch das Infusionsgerät, einer Nutzungsmetrik des Infusionssets, nachdem das Infusionsset mit der Pumpe gekoppelt wurde.

11. Nicht-flüchtiges maschinenlesbares Medium nach einem der Ansprüche 8 bis 10, wobei das Empfangen der Arzneimittelidentifikation an dem Infusionsgerät das Empfangen manuell eingegebener Daten umfasst.

12. Nicht-flüchtiges maschinenlesbares Medium nach einem der Ansprüche 8 bis 11, wobei das Empfangen der Arzneimittelidentifikation an dem Infusionsgerät das Empfangen der Arzneimittelidentifikation aus einem Infusionsauftrag umfasst, der von dem System zum Speichern und Abrufen elektronischer Krankenakten an das Infusionsgerät gesendet wird.

13. Nicht-flüchtiges maschinenlesbares Medium nach einem der Ansprüche 8 bis 12, wobei das Infusionsgerät dazu ausgebildet ist, eine Mehrzahl von Infusionsaufträgen zu speichern und Infusionsparameter, die einem jeweiligen der mehreren Infusionsaufträge betreffend das medizinische Fluid zugeordnet sind, automatisch zu der Pumpe herunterzuladen, mit der das Infusionsset gekoppelt ist.

14. Nicht-flüchtiges maschinenlesbares Medium nach einem der Ansprüche 8 bis 13, wobei das Übermitteln der Infusionsset-Kennung erfolgt, nachdem das Infusionsset mit der Pumpe gekoppelt wurde, und das System zum Speichern und Abrufen elektronischer Krankenakten Infusionsauftragsinformationen an die Pumpe sendet, mit der das Infusionsset gekoppelt ist, nachdem bestimmt wurde, dass die Arzneimittelinformation der Arzneimittelidentifikation entspricht.

## Revendications

1. Système de perfusion (200), comprenant : un récepteur de set de perfusion (246) ; une pompe (202) agencée pour appliquer une force à au moins une partie d'un tube d'un set de perfusion après que le set de perfusion a été reçu par le récepteur de set de perfusion, afin de pomper un fluide à travers le set de perfusion ; un émetteur-récepteur de données de réseau (206) ; et un processeur configuré pour : détecter (306), par le récepteur de set de perfusion, un identifiant de set de perfusion pour le set de perfusion, transmis par un module de communication inclus dans le set de perfusion lorsque le set de perfusion est chargé dans la pompe ; transmettre (310), via l'émetteur-récepteur de données de réseau, au système de stockage et de récupération de dossiers médicaux électroniques, l'identifiant de set de perfusion ; recevoir (310), via l'émetteur-récepteur de données de réseau, en provenance du système de stockage et de récupération de dossiers médicaux électroniques, une information médicamenteuse concernant un contenu d'un contenant de perfusion associé à l'identifiant de set de perfusion ; recevoir (308), au niveau de la pompe, séparément de l'information médicamenteuse reçue, une identification du médicament pour un fluide médical devant être perfusé par la pompe ; déterminer si l'information médicamenteuse reçue correspond à l'identification du médicament reçue ; et autoriser (312) la pompe à démarrer une perfusion du fluide médical lorsque l'information médicamenteuse reçue correspond à l'identification du médicament reçue, et empêcher (314) la pompe de démarrer la perfusion lorsque l'information médicamenteuse reçue ne correspond pas à l'identification du médicament reçue.

2. Système de perfusion selon la revendication 1, dans lequel la détection de l'identifiant de set de perfusion comprend : l'activation d'un capteur ; et la réception, depuis le capteur, d'une valeur détectée au niveau du set de perfusion.

3. Système de perfusion selon la revendication 1 ou 2, dans lequel le fait que le processeur soit configuré pour recevoir l'identification du médicament comprend le fait que le processeur soit configuré pour extraire l'identification du médicament d'un message de demande de programmation automatique envoyé à la pompe par un système de réseau de données.

4. Système de perfusion selon la revendication 3, dans lequel la pompe reçoit plusieurs messages de demande de programmation automatique, et dans lequel le processeur est configuré pour : recevoir un ordre de perfusion en provenance du système de stockage et de récupération de dossiers médicaux électroniques ; et déterminer, à partir de l'identifiant de set de perfusion, lequel des plusieurs messages de demande de programmation automatique contient un ordre de perfusion qui correspond à l'identifiant de set de perfusion.

5. Système de perfusion selon l'une quelconque des revendications 1 à 4, dans lequel le fait d'autoriser la pompe à démarrer la perfusion comprend la présentation, via un écran du système de perfusion, d'un élément de commande pour activer la pompe afin de délivrer le contenu du contenant de perfusion.

6. Système de perfusion selon l'une quelconque des revendications 1 à 5, dans lequel le processeur est configuré pour : générer une métrique d'utilisation pour le set de perfusion basée au moins en partie sur un moment auquel la pompe commence à perfuser le contenu.

7. Système de perfusion selon la revendication 6, dans lequel le processeur est configuré pour : détecter une non-conformité de la métrique d'utilisation par rapport à un seuil de sécurité ; et déclencher une alarme pour alerter un soignant lorsque la métrique d'utilisation indique que le set de perfusion a été utilisé pendant une période dépassant un seuil de sécurité.

8. Support non transitoire lisible par machine incorporant des instructions qui, lorsqu'elles sont exécutées par une machine de traitement de données, amènent la machine à exécuter un procédé d'association d'un contenant de perfusion, d'un set de perfusion et d'une pompe d'un dispositif de perfusion, le procédé comprenant : la réception, au niveau d'un dispositif de perfusion, d'une indication selon laquelle un set de perfusion est en cours de couplage à une pompe, le set de perfusion comprenant un tube sur lequel une force est appliquée par la pompe pour pomper un fluide à travers le set de perfusion ; la détection, par un récepteur de set de perfusion du dispositif de perfusion, d'un identifiant de set de perfusion du set de perfusion, l'identifiant de set de perfusion étant transmis par un module de communication inclus dans le set de perfusion lorsque le set de perfusion est couplé à la pompe ; la transmission, au système de stockage et de récupération de dossiers médicaux électroniques, de l'identifiant de set de perfusion ; la réception, en provenance du système de stockage et de récupération de dossiers médicaux électroniques, d'une information médicamenteuse concernant un contenu d'un contenant de perfusion associé à l'identifiant de set de perfusion ; la réception, au niveau du dispositif de perfusion, séparément de l'information médicamenteuse reçue, d'une identification du médicament pour un fluide médical devant être perfusé ; l'évaluation, au niveau du dispositif de perfusion, de l'identification du médicament par rapport à l'information médicamenteuse ; la détermination, sur la base de l'évaluation, si l'information médicamenteuse correspond à l'identification du médicament ; lorsqu'il est déterminé que l'information médicamenteuse correspond à l'identification du médicament, l'autorisation de la pompe à démarrer une perfusion du fluide médical ; et lorsqu'il est déterminé que l'information médicamenteuse ne correspond pas à l'identification du médicament pour le fluide médical reçu au niveau de la pompe : l'empêchement de la pompe de démarrer la perfusion ; et la génération d'une alerte indiquant qu'un contenant de perfusion pour le fluide médical ne correspond pas à l'identification du médicament reçue au niveau du dispositif de perfusion.

9. Support non transitoire lisible par machine selon la revendication 8, dans lequel le système de stockage et de récupération de dossiers médicaux électroniques reçoit et stocke l'association d'un identifiant de contenant de perfusion avec l'identifiant de set de perfusion.

10. Support non transitoire lisible par machine selon la revendication 8 ou la revendication 9, le procédé comprenant en outre la surveillance, par le dispositif de perfusion, d'une métrique d'utilisation du set de perfusion après que le set de perfusion a été couplé à la pompe.

11. Support non transitoire lisible par machine selon l'une quelconque des revendications 8 à 10, dans lequel la réception, au niveau du dispositif de perfusion, de l'identification du médicament comprend la réception de données saisies manuellement.

12. Support non transitoire lisible par machine selon l'une quelconque des revendications 8 à 11, dans lequel la réception, au niveau du dispositif de perfusion, de l'identification du médicament comprend la réception de l'identification du médicament à partir d'un ordre de perfusion envoyé au dispositif de perfusion par le système de stockage et de récupération de dossiers médicaux électroniques.

13. Support non transitoire lisible par machine selon l'une quelconque des revendications 8 à 12, dans lequel le dispositif de perfusion est configuré pour stocker une pluralité d'ordres de perfusion et télécharger automatiquement des paramètres de perfusion associés à un ordre respectif parmi la pluralité d'ordres de perfusion concernant le fluide médical vers la pompe à laquelle le set de perfusion est couplé.

14. Support non transitoire lisible par machine selon l'une quelconque des revendications 8 à 13, dans lequel la transmission de l'identifiant de set de perfusion intervient après que le set de perfusion a été couplé à la pompe, et le système de stockage et de récupération de dossiers médicaux électroniques envoie des informations d'ordre de perfusion à la pompe à laquelle le set de perfusion est couplé, après avoir déterminé que l'information médicamenteuse correspond à l'identification du médicament.
